Europäisches Patentamt

European Patent Office

Office européen des brevets

(19)

(11) Publication number: **0 384 573**

**A1**

# EUROPEAN PATENT APPLICATION

(21) Application number: **90300641.9**

(22) Date of filing: **22.01.90**

(51) Int. Cl.5: **C08B 11/20, C08B 37/00, A61K 9/36**

(30) Priority: **23.01.89 US 300666**

(43) Date of publication of application:
**29.08.90 Bulletin 90/35**

(84) Designated Contracting States:
**BE DE FR GB IT NL SE**

(71) Applicant: **THE DOW CHEMICAL COMPANY**
**2030 Dow Center Abbott Road**
**Midland, MI 48640(US)**

(72) Inventor: **Christensen, Stephen B.**
**4410 Ottawa**
**Midland, Michigan 48640(US)**
Inventor: **Schulz, Gary J.**
**81 Oaklawn**
**Midland, Michigan 48640(US)**
Inventor: **Kling, Susan**
**1661 W. Chippewa River**
**Midland, Michigan 48640(US)**

(74) Representative: **Burford, Anthony Frederick et al**
**W.H. Beck, Greener & Co. 7 Stone Buildings**
**Lincoln's Inn**
**London WC2A 3SZ(GB)**

(54) **Removal of color material from water-soluble polymer solutions.**

(57) Color material is removed from aqueous solutions of water-soluble polysaccharide ether, e.g. cellulose ethers, by contact with an anion exchange resin. Preferred anion exchange resins are derived from epoxy-amine polymers, acrylic-divinylbenzene copolymer or especially styrene-divinylbenzene copolymer.

EP 0 384 573 A1

## REMOVAL OF COLOR MATERIAL FROM WATER-SOLUBLE POLYMER SOLUTIONS

Water-soluble polymers are used for their many different properties and are employed in a variety of applications. The polymers are used as thickeners, binders, film formers, water retention aids, suspension aids, surfactants, lubricants, protective colloids, emulsifiers and the like, and are used in applications such as foods, cosmetics, pharmaceuticals, latex paints, construction products, ceramics and a host of other applications. A problem, encountered by those that produce and consequently those that use the water-soluble polymers, is the presence of contaminants, such as undesirable color and salt. The undesirable color contamination is of particular concern to the pharmaceutical industry.

The color in water-soluble polymers and, in particular, cellulose ether solutions can vary from a very pale yellow to a golden or even brown hue, depending on the concentration of the solution and the amount of color bodies in the product. For some applications the presence of this color is not a problem, but for other applications, in particular tablet coating in the pharmaceutical industry, the colorant causes difficulties. It would be desirable to have a method of removing or reducing the residual color for those applications in which low levels are required.

This invention is a process for removing the undesirable residual color material from water-soluble polymers by contacting an aqueous solution of such polymers with anion exchange resins.

This invention can be used to decolorize, or reduce the color of aqueous solutions of water-soluble polymers comprising polysaccharide ethers, such as cellulose ethers. In particular the invention has proven useful in decolorizing solutions that are used in the pharmaceutical industry. The process can also be used to decolorize water-soluble polymeric material that has been recovered by ultrafiltration of process wash streams during the manufacture of the water-soluble polymers. Said recovered solutions are typically highly colored and some decolorization is necessary to produce an acceptable product. Additionally, this invention can be useful in removing other undesirable material from water-soluble polymer solutions, such as minor amounts of salt.

Water-soluble polymers that are of particular interest in the present invention comprise water-soluble polysaccharide ethers. Polysaccharide ethers are the etherified products of a polysaccharide. Representative polysaccharides include: cellulose; natural gums such as arabic, xanthan, and guar; dextran; and starch. Cellulose is the preferred polysaccharide. Examples of such ethers include cellulose ethers such as alkylcellulose or hydroxyalkyl alkylcellulose, methylcellulose, hydroxypropyl methylcellulose, hydroxyethyl methylcellulose, ethyl methylcellulose, hydroxyethyl hydroxypropyl methylcellulose, hydroxyethylcellulose, and hydroxypropylcellulose.

Anion exchange resins that are useful in the invention herein are polymeric materials that have a positively charged matrix and exchangeable negative ions or anions, typically functionalized copolymer beads or copolymer beads that have been pulverized. Preferably, the anion exchange resin is employed in the bead form of the resin. The polymer beads from which anion exchange resins are derived and can be prepared from include epoxy resin-based polymers, (e.g. an epoxy-amine polymer); acrylic copolymers (e.g. an acrylic-divinylbenzene copolymer); and copolymer compositions of styrene-divinylbenzene. Anionic exchange resins are typically prepared via suspension polymerization of comonomers and are then functionalized with groups that can exchange anions. To functionalize the copolymer beads, the beads can be chloromethylated with chloromethyl methylether (CMME) and then aminated with a dimethylamine to provide weak base functionality or a trimethylamine to provide a strong base functionality.

Another useful resin which is employed in the invention herein is an adsorptive, porous, post-crosslinked resin in bead form. The adsorptive, porous post-crosslinked resin is prepared by contacting a functionalized resin in a swollen state with a Friedel-Craft catalyst under conditions effective to catalyze the post-crosslinking and rearrangement of the swollen functionalized resin. Examples of these polymeric post crosslinked absorbent resins are taught in U.S. Patent 4,263,407.

The anion exchange resin useful in the invention herein typically has a substituent that is either a strong-base quaternary ammonium group or a weak-base amino group. If the substituent is an alkyl quaternary ammonium salt, the material is known in the industry as a strong-base anion exchange resin. If the substituent is an amine or alkyl amine group, the material is known as a weak-base resin. The weak-base resins are effective in the unaltered or free-base form. The strong-base resin is effective with a chloride or hydroxide counterion, also known in the ion exchange art as the chloride or hydroxide ion form.

It has also been found useful to use the ammonium salt form of the weak-base resin which is formed by the interaction of a weak-base resin with any suitable mineral acid, such as hydrochloric acid and sulfuric acid, with hydrochloric acid being preferred.

While any anion exchange resin will serve to reduce the color of the aqueous solutions, preferably the resin has a large internal pore size. Thus generally, a macroporous resin is to be preferred over a gel-type resin. Generally, suitable resins usually have a styrene-divinylbenzene matrix, such as DOWEX® SBR-P (a Trademark of the Dow Chemical Company) a strong-base gel resin, DOWEX® 11 a strong-base gel resin, DOWEX® MWA-1 a weak-base macroporous resin, and DOWEX® MSA-1 a strong-base macroporous resin.

Other suitable resins include the following resins: a resin having a styrene-divinylbenzene matrix, e.g. AMBERLITE® IRA-93 (a Trademark of Rohm Haas Company) a weak-base macroreticular styrene-divinylbenzene resin; a acrylic-divinylbenzene matrix, e.g. AMBERLITE® IRA-958 a strong-base macroreticular acrylic-divinylbenzene resin; an epoxy resin-based polymer, e.g. DOWEX® WGR-2 an intermediate base resin with an epoxy amine matrix. Of course there are many equivalents for all of these resins. The preferred resins of this invention are the DOWEX® MWA-1 weak-base macroporous resin and the DOWEX® MSA-1 strong-base macroporous resin.

In addition to using particular anionic resins, some of which are described hereinabove, mixed bed resins can also be useful in the invention. Such a suitable resin is DOWEX® MR-3, a combination of a strong-base gel styrene-divinylbenzene resin and a strong acid cation exchange styrene-divinylbenzene resin.

If the anion exchange resin is a strong-base resin or the ammonium salt of a weak-base resin, then a counterion or anion will be present in the system. This counterion may be a chloride, hydroxide, or sulfate anion. In decolorizing solutions in which high pH levels are to be avoided, chloride is the preferred anion. The chloride ion is preferred since it is most commonly found in these water-soluble polymer solutions. Use of a strong-base resin with the hydroxide counterion, in situations where demineralization as well as color removal are desired, allows the hydroxide counterion in the anion exchange column to also function as one half of a two-stage desalting process. Such an arrangement eliminates the need for an additional anion exchange column.

To remove the undesirable material from the aqueous solution, e.g., color, salt and the like, a column is first packed with an anion exchange resin and then washed with water. If necessary, the column may then be converted into the proper anionic form by treatment with the appropriate solution. For instance, pretreatment of a strong-base resin could be performed by passing any of the following solutions down the column: 4 percent sodium hydroxide solution to give the hydroxide form of the resin; 15 percent sodium chloride solution to give the chloride form of the resin; 6 percent hydrochloric acid solution to give the chloride form of the resin; 4 percent sulfuric acid to give the sulfate form of the resin.

To convert the column to the proper ionic form using a weak-base resin the following solutions, for example, could be washed down the column: 6 percent hydrochloric acid solution to give the ammonium chloride salt form of the resin; or 4 percent sulfuric acid solution to give the ammonium sulfate salt form of the resin. These values are given only as examples and are not meant to be limiting. The column is then washed with water to remove excess ions. Finally, the water-soluble polymer solution is passed down the column at such a flow rate that the desired level of decolorization is achieved.

The flow rate of the process and the size of the column to be used are a function of: the amount of color in the solution, the resin being used and the amount of decolorization desired. The exact values for each of these parameters must be adjusted to meet the desired results. In general, the amount of decolorization is a function of the contact time of the solution with the resin, with longer contact times given to more highly decolorized solutions. For a given amount of decolorization, the required contact time can be achieved by numerous combinations of flow rate and column size. Typically, the flow rates range from 0.001 to 10 gpm/sg ft. (0.04 to 400 liters/min•m²), preferably from 0.1 to 1.5 gpm/sg ft (4 to 60 liters/min. m²).

The process is typically operated at ambient temperatures of 15° to 35°C. Elevated temperatures of 50° to 60°C are effective, but at higher temperatures some of the water-soluble polymer may precipitate or gel. This gelling or precipitation could cause the column to be blocked, thereby inhibiting the flow of the aqueous solution through the column. In instances where one would not be concerned with the water-soluble polymer gelling, elevated temperatures are workable.

The water-soluble polymer solution may contain any of the aforementioned etherified polysaccharides, with the main concerns being the viscosity, pH, and salt content of the solution. The solution should not be so viscous that reasonable flow rates as described herein cannot be achieved without resorting to excess column pressures to force the aqueous solution through the column. Typically accepted pressures to aid in the process are 5 to 300 psig (140 to 2200 kPa absolute), preferably from 40 to 80 psig (380 to 650 kPa absolute).

The pH of the solution is also a factor in the efficiency of the process. It has been found that if the pH of the solution is too low the efficiency of the process could be reduced. Additionally, if the resin being used is the ammonium salt of a weak-base resin it is advantageous to keep the pH below about 7 to avoid a rapid deprotonation of the resin by the water-soluble polymer solution. The preferred pH of the solution is therefore generally between 4 and 7.

The salt content of the solution must be low enough so that the salt does not interfere with the decolorization process. Salt contents as high as 1 to 2 weight percent are acceptable, with the preferred value being anything below this level. Moreover, this process can be employed to remove minor amounts of salt contained in the water-soluble polymer solution.

When the column's ability to remove the undesirable material is exhausted or at least the removal is not at the desirable level, then the resin in the column can be regenerated and reused. The first step in the regeneration is to wash the column with water. The column is then treated with any of a variety of agents having the properties of removing accumulated color bodies from the resin and restoring the activity of the resin to an effective level. For example, if the undesirable materials are color bodies in a cellulose ether solution, a salt solution of 10 to 26 weight percent, or either a sodium hydroxide or a mineral acid solution of 1 to 10 weight percent, preferably 2 to 4 weight percent, can be used to release the accumulated bodies from the column. The preferred regeneration step is to treat the column with a mineral acid, in particular hydrochloric acid. This has the advantage of both regenerating the column and in those cases where a weak-base resin is being used, the acid serves to repronate the resin before the next cycle.

Typically to regenerate the column using the acid, two bed volumes of 1 to 10 weight percent hydrochloric acid are pumped through a column over a period of time sufficient to restore the column's efficiency, with 4 to 6 weight percent being preferred. This can take from 45 minutes to 2 hours. The acid is then purged from the column with fresh water and the column is ready for use again. The regeneration step is normally performed at an ambient temperature. Alternatively, elevated temperatures can be used as long as the temperature used does not exceed the upper-temperature limitation of the resin that is employed in the process.

Examples

Example 1 - Use of Anion Exchange Resins to Remove Color from Cellulose Ether Solutions:

A 2.5 centimeter (cm) by 30 cm column is packed with resin and used to purify a 10 weight percent, crude hydroxypropyl methylcellulose solution. This crude polymer solution has at 2 weight percent, a viscosity of about 5 cps (mPa•s). The color of this crude solution, as measured on a Hunter Lab Model D25 Optical Sensor, is a value of 48 APHA units (American Public Health Association) for a filtered 2 weight percent solution at pH 7. APHA units are determined via use of the Hunter Lab Model D25 Optical Sensor and analysis standards published in "Standards Method Manual: Physical, Chemical, Biological analysis of Water-Waste Water", 16th ed., jointly published by the American Public Health Association, the American Water Works Association, and the Water Pollution Control Federation. With this instrument the lower the numerical value the less color that is present in the solution. For instance, distilled water has a numerical value of 0.

The procedure as herein described above is carried out for each of several anion exchange resin sample, A, B, C, ... J. For each of the samples, 120 mL of a resin is placed in the column, then the resin is washed with about two bed volumes (one bed volume equals 120 mL) of water. Any additional column treatments are noted below. The polymer solution is then pumped through the column at a flow rate of about 0.27 gpm/ft$^2$ (11 liters/min•m$^2$). After one hour a sample is collected for 20 minutes. This sample is diluted to a 2 weight percent solution, filtered, adjusted to a pH of 7 and measured for color.

A. DOWEX® MWA-1, weak-base macroporous, styrene-divinylbenzene resin is used in the free-base form.

B. DOWEX® MWA-1, weak-base macroporous, styrene-divinylbenzene resin is used in the ammonium chloride salt form. Before packing the column with the resin, the salt form is made by slurrying the dry resin with 6 percent hydrochloric acid.

C. DOWEX® MSA-1, strong-base macroporous styrene-divinylbenzene resin is used in the hydroxide counterion form. The counterionic form is made by washing the column with 3 bed volumes of 4 percent sodium hydroxide, followed by 3 bed volumes of water.

D. DOWEX® MSA-1, strong-base macroporous styrene-divinylbenzene resin is used in the sulfate counterion form. The counterion form is made by washing column with 3 bed volumes of 6 percent sulfuric acid, followed by 3 bed volumes of water.

E. DOWEX® MSA-1, strong-base macroporous styrene-divinylbenzene resin is used in the chloride counterion form. The resin is received in the counterion form and is employed as received.

F. AMBERLITE® IRA-93 (a Trademark of Rohm Haas Company), weak-base macroreticular styrene-divinylbenzene resin is used in the ammonium chloride salt form. Before packing the column with the resin, the salt form is made by slurrying the dry resin with 6 percent hydrochloric acid.

G. DOWEX® 11, strong-base gel styrene-divinylbenzene resin is used in the chloride counterion form. The resin is received in the counterion form and is employed as received.

H. AMBERLITE® IRA-958, strong-base macroreticular acrylic-divinylbenzene resin is used in the chloride counterion form. The resin is received in the counterion form and is employed as received.

I. DOWEX® MR-3, mixed resin bed containing DOWEX® SBR,strong-base gel styrene-divinylbenzene resin used in the hydroxide counterion form, and DOWEX® HCR-S, strong acid cation exchange styrene-divinylbenzene resin in the hydrogen counterion form. The resin is received in the counterion form and is employed as received.

J. DOWEX® WGR-2, intermediate base, epoxy amine resin used in the ammonium chloride salt form. Before packing the column with the resin, the salt form is made by slurrying the dry resin with 6 percent hydrochloric acid.

| Samples | Color APHA units* |
|---|---|
| A | 19 |
| B | 19 |
| C | 19 |
| D | 21 |
| E | 22 |
| F | 23 |
| G | 24 |
| H | 24 |
| I | 25 |
| J | 33 |
| Crude Cellulose Ether Solution | 48 |
| Distilled Water | 0 |

*Color value as measured by a Hunter Lab Model D25 Optical Sensor. The lower the numerical value the less color that is present in the solution.

Results

As is seen in the above table, by running the crude cellulose ether solution down the ion exchange column containing anion exchange resins, colored bodies are removed from the solution. For example, before running the cellulose ether solution down the column, the crude solution has a color value of 48 APHA units; whereas, a sample which has gone through the column containing DOWEX® MWA-1 anion exchange resins has a color value of 19 APHA units (Sample A).

Example 2 - Use of Anion Exchange Resins to Remove Color From Wash Water of Cellulose Ether Solutions

A 2 inch (5 cm) diameter x 30 feet (9.1 meters) long column is filled with Dowex Macroporous MWA-1 resin. The column is backflushed with 50 lbs. (22.7 kg) of deionized water. The resin is protonated with 40 lbs (18.2 kg) of 6 percent HCL at about 22 lbs/hr (10 kg/hr) in the up-flow direction. The acid is purged with 50 lbs (22.7 kg) water at about 22 lbs/hr (10 kg/hr) in the up-flow direction. Then, a 13 percent cellulose ether solution, that has a viscosity of 1.6 cps (mPa•s) as a 2 percent solution, is recovered from process wash streams by ultrafiltration. The recovered cellulose ether solution is adjusted to a pH value of 5.3 with concentrated HCL. The recovered cellulose ether solution is fed to the column at 1.3 gpm/sq ft (53.0 liters/min•m²) (down-flow).

After 110 lbs (50 kg) of solution are pumped through the column, the column is purged with 50 lbs (22.7 kg) water, is regenerated with 40 lbs (18.2 kg) of 6 percent HCl and then purged with 50 more lbs (22.7 kg) water (similar to initial conditioning). The column is used again to remove color. This sequence is repeated several times with no loss of column capacity. This processing is done at about 25° C.

## Results

After the crude cellulose ether solution is run down the column, a sample is taken. The sample is measured using a Bausch & Lomb Spectronic 20 at 490 nanometers. In the recovered sample, 93 percent of the color bodies were removed from the solution.

## Example 3 - Use of Anion Exchange Resins to Remove Color from Cellulose Ether Solutions (Scale-up version of Example 1):

Using the same ion exchange resin apparatus as in Example No. 2, 1300 gms of Dowex MWA-1 resin is loaded into the column. The column is backflushed with about 5 gallons (18.9 liters) of deionized water. The resin is protonated in the backflush mode with 40 lbs (18.2 kg) of 6 percent HCl. The HCl is slowly pumped through the column in order to provide residence time for the pronation of the resin. The residual acid is purged with water.

A 9.1 percent solution of hydroxypropyl methylcellulose, having a viscosity of about 3.5 cps (mPa•s) as a 2 percent solution is prepared by adding 25 lbs (11.4 kg) of hydroxypropyl methylcellulose to 225 lbs (102 kg) of deionized water and then agitated for 3 hrs to form a clear solution. This solution is fed to the above prepared column at 1.11 gpm/sq ft (45.2 liters/min. m²) in the down flow direction. A sample of the hydroxypropyl methylcellulose solution is collected after the solution is passed through the column.

After the hydroxypropyl methylcellulose solution is pumped through the column, the column is regenerated. The column is purged with 50 lbs (22.7 kg) of water, and 40 lbs (18.2 kg) of 6 percent HCl is pumped through the column in the upflow direction. The acid is purged from the column in the upflow direction with 50 lbs (22.7 kg) of water. The column is ready for use again. This process was done at about 25° C.

## Results

A sample of the hydroxypropyl methylcellulose solution is collected. After the sample has run through the anion exchange column, the color value is 14.7 APHA units. Before running the hydroxypropyl methylcellulose solution through the column, the solution had a color value of 60 APHA units as measured with a Hunter Colorimeter.

## Claims

1. A method for removing color bodies from an aqueous solution containing a water-soluble polysaccharide ether, comprising contacting the water-soluble polymeric solution with an anion exchange resin.

2. A method as claimed in Claim 1, wherein the anion exchange resin is in bead form.

3. A method as claimed in any one of the preceding claims, wherein the anion exchange resin is a macroporous resin.

4. A method as claimed in any one of the prece ing claims, wherein the pH of the solution is pH 4 to pH 7.

5. A method as claimed in any one of the preceding claims, wherein the anion exchange resin is dreived from an epoxy-amine polymer or an acrylic-divinylbenzene copolymer.

6. A method as claimed in any one of Claims 1 to 4 wherein the anion exchange resin is derived from a styrene-divinylbenzene copolymer.

7. A method as claimed in any one of the preceding claims wherein the anion exchange resin is a weak-base resin in ammonium salt form.

8. A method as claimed in any one of Claims 1 to 6, wherein the anion exchange resin is a strong-base resin in chloride or hydroxide form.

9. A method as claimed in Claim 6, wherein the anion exchange resin is a strong-base styrene-divinylbenzene gel-type resin in a mixed bed with a strong-acid cation exchange styrene-divinylbenzene resin.

10. A method as claimed in any one of the preceding claims, wherein the water-soluble polysaccharide ether is a cellulose ether.

11. The use for tablet coating of an aqueous water-soluble polysaccharide ether solution decolorized by a method as claimed in any one of the preceding claims.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-2 617 800 (W.E. BERGMAN) <br> * Claim 1 * <br> --- | 1 | C 08 B 11/20 <br> C 08 B 37/00 <br> A 61 K 9/36 |
| Y | CHEMICAL ABSTRACTS, vol. 104, no. 8, 24th February 1986, page 122, abstract no. 52891b, Columbus, Ohio, US; & JP-A-60 106 539 (ITOCHU SEITO K.K.) 12-06-1985 <br> * Abstract * <br> --- | 1 | |
| A | GB-A- 736 304 (HERCULES POWDER) <br> * Claim 1 * <br> --- | | |
| A | EP-A-0 194 877 (DOW) <br> * Abstract; page 1 * <br> ----- | | |

TECHNICAL FIELDS
SEARCHED (Int. Cl.5)

C 08 B

The present search report has been drawn up for all claims

| Place of search | Date of completion of the search | Examiner |
|---|---|---|
| THE HAGUE | 20-04-1990 | SOMERVILLE F.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

               

& : member of the same patent family, corresponding document

EPO FORM 1503 03.82 (P0401)